# EUROPEAN PATENT APPLICATION

(11) **EP 0 619 317 A1**
(43) Date of publication of application: **12.10.1994**
(21) Application number: 93400914.3
(22) Date of filing: 07.04.1993
(51) Int. Cl.: C07H 15/04, C07H 15/14

(54) **An expeditious synthesis of C-disaccharides using a temporary ketal connection**

(71) Applicant: CENTRE NATIONAL DE LA RECHERCHE SCIENTIFIQUE, 75007 Paris (FR)
(72) Inventor: Sinäy, Pierre, F-75009 Paris (FR); Mallet, Jean-Maurice, F-75013 Paris (FR); Vauzeilles, Boris, F-92160 Antony (FR)
(74) Representative: Gutmann, Ernest

(57) **Abstract**

The present invention relates to a rapid method for synthesizing C-disaccharides. More particularly, the process involves the formation of a tethered ketal intermediate which is then cyclized and detethered resulting in a protected C-disaccharide as a single diastereomer.

## Description

### BACKGROUND OF THE INVENTION

The present invention relates to a process for synthesizing disaccharides in which the interglycosidic oxygen atom linkage is replaced by a methylene group. More specifically, the present invention relates to an expeditious process to synthesize C-disaccharides and other types of carbon-linked disaccharides by use of a temporary ketal tether to facilitate carbon-carbon bond formation.

### Description of the Prior Art

C-disaccharides constitute a class of non-natural analogs of disaccharides in which the interglycosidic oxygen atom is replaced by a methylene group. These C-disaccharides are conformationally close to their natural counterparts but are not degraded by chemical and/or biological hydrolysis. As potential inhibitors of glycosyl hydrolases such as the disaccharides of the digestive tract as described by Martin et al J. Org. Chem., 55, 5188 (1990) and as probes of the stereoelectronic effects which may control the conformation of oligosaccharides as described by Lai, W. PH.D. Dissertation, SUNY-Binghamton (1992) these pseudodisaccharides are of considerable interest.

Furthermore, the great interest in natural and unnatural glycosidase inhibitors has even increased because HIV activity might be affected by such compounds. See, Gruters et al., Biochem. Biophys. Res. Commun., 148, 206 (1983). Furthermore, the inaccessibility to hydrolytic cleavage of the C-disaccharides is a particular stimulus to their synthesis.

Rapid progress has also been observed in the past for the selective synthesis of complex oligosaccharides which has stimulated the understanding of the cellular biological role that is contributed to these compounds.

The first synthesis of a C-disaccharide was reported in 1983 by Sinay et al. J. Chem. Soc. Chem. Commun., 1353 (1983). A β(1-->6)-methylene bridged gentiobiose derivative was synthesized by using a C₆-electrophile, C₇-nucleophile course and required diastereocontrol only at the C₆-species. In this method 2, 3, 4, 6-tetrabenzylglucopyranolactone was condensed with the anion of methyl 6, 7-dideoxy-2,3,4-tri-O-benzyl-α-1-gluco-hept-6-ynopyranoside followed by triethylsilane reduction. After hydrogenolysis, a synthetic β(1-->6)-C-disaccharide was obtained.

After the first synthesis of a C-disaccharide was described in the art, many different scientific groups proceeded to work on different methods for the synthesis of a variety of different C-disaccharides in different stereo specific configurations. For example, Giese et al., Angew Chem., 98, 459 (1986); Angew Chem., Int. Ed. Engl., 25, 450 (1986); and Tetrahedron Lett. , 29, 1375 (1988) described the addition of glycosyl radicals to sugar 2-methylene lactones which lead preferentially to α(1-->2)-connection with 2,3-trans stereochemistry in the former lactone species.

Kishi et al. in J. Org. Chem., 52, 1370 (1987); Goekjian et al., ibid 52, 4823 (1987); Babirad et al., ibid 52, 4825 (1987); Wang et al., ibid 53, 4151 (1988); Miller et al., ibid 53, 5580 (1988); and Dyer et al., ibid 53, 3383 (1988) used a combination of C₅-electrophile + C₈-nucleophile species to synthesize the compounds of the following formula: 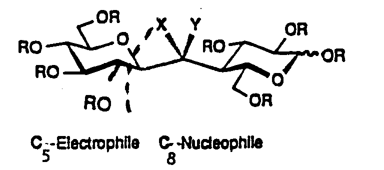
wherein X=Y=H. Moreover, Haneda et al. in J. Org. Chem. 57, 490 (1992) have described the synthesis of a C,C-trisaccharide which is the bis-carba-analog of a blood group antigenic determinant.

Preuss et al. in J. Carbohydr. Chem., 10, 887 (1991) also describe the synthesis of methylene bridged C-disaccharides having the following formula: 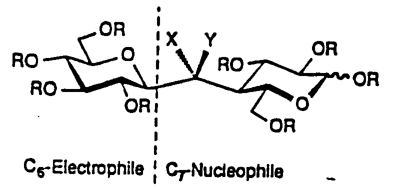
wherein X=Y=H via a C₆-electrophile + C₇-nucleophile route.

Martin et al. J. Org. Chem., 58, 176-185 (1993) recently describe the synthesis of β-(1--->6)-linked C-disaccharide based on the fluoride ion-mediated coupling of the base stable nitronate anion derived from glycosylnitromethane and an aldehydo-hexodialdose or hexose derivative.

Furthermore, all of the prior art processes teach only a 5% to 20% overall yield for the synthesis of C-disaccharides.

Although the above-described methods are indeed feasible for the synthesis of various C-disaccharides, a simple and expeditious method is still needed in the art to synthesize these various non-natural analogs for a multitude of purposes, especially for the synthesis of C-disaccharides or C-oligosaccharides for various pharmaceuticals and for the synthesis of higher sugars.

It has now been discovered that an expeditious process can be used to synthesize C-disaccharides using a temporary ketal connection or tether. Furthermore, the use of tethering and the removal of the ketal connection provides a process in which the overall yield of C-disaccharides is about 40%.

### OBJECTS AND SUMMARY OF THE INVENTION

Thus, it is an object of the present invention to avoid or alleviate the problems associated with the prior art.

It is another object of the invention to provide a process which avoids a multistep process for the synthesis of C-disaccharides and related compounds which is regioselective and exceptionally expeditious.

It is yet another object of the present invention to provide a means to form a C-disaccharide via a ketal tether to facilitate carbon-carbon bond formation of the two starting compounds.

It is a further object of the present invention to provide a 9 endo-trig radical cyclization process for synthesis of C-disaccharides which provides a higher yield of the C-disaccharide.

It is a further object of the present invention to provide a process for the synthesis of C-disaccharides said process comprises the steps of:
(a) synthesizing a glycosyl donor wherein said glycosyl donor contains a vinyl ether attached to a glycosyl moiety;
(b) condensing said glycosyl donor with a glycosyl acceptor having an exomethylene group attached to a glycosyl moiety on said glycosyl acceptor to form a temporary ketal;
(c) cyclizing said temporary ketal;
(d) detethering said cyclized temporary ketal to form a protected C-disaccharide; and
(e) debenzylating said protected C-disaccharide to form a methyl α-C-disaccharide.

A preferred embodiment of the present invention features a process for the synthesis of C-disaccharides, said process comprising the steps of:
(a) synthesizing a glycosyl donor of phenyl 2-O-(methyl-vinyl)-3,4,6-tri-O-benzyl-1-seleno- α-D-mannopyranoside from 3,4,6-tri-O-benzyl-1, 2-0-(methoxy ethyl)-β-D-mannopyranose;
(b) condensing said glycosyl donor of phenyl-2-O-(methyl-vinyl)-3,4,6-tri-O-benzyl-1- seleno-α-D-mannopyranoside with a solution of methyl-2,3-di-O-benzyl-4-deoxy-4-6-methylene-α-D- xylohexopyranoside to form a tethered ketal intermediate;
(c) cyclizing said tethered ketal intermediate;
(d) detethering and acetylating said cyclized tethered ketal intermediate to form methyl 6-O-acetyl-2,3-di-O-benzyl-4-O-(2-O-acetyl-3,4,6-tri-O-benzyl-α-D-mannopyranosyl-methyl)-α-D-glucopyranoside; and
(e) de-acetylating and debenzylating methyl 6-O-acetyl-2, 3-di-O-benzyl-4-O-(2-O-acetyl-3, 4, 6-tri-O- benzyl-α-D-mannopyranosyl-methyl)-α-D-glucopyranoside to form methyl 4-O-(α-D-mannopyranosyl-methyl)-α-D-glucopyranoside.

### BRIEF DESCRIPTION OF THE DRAWINGS

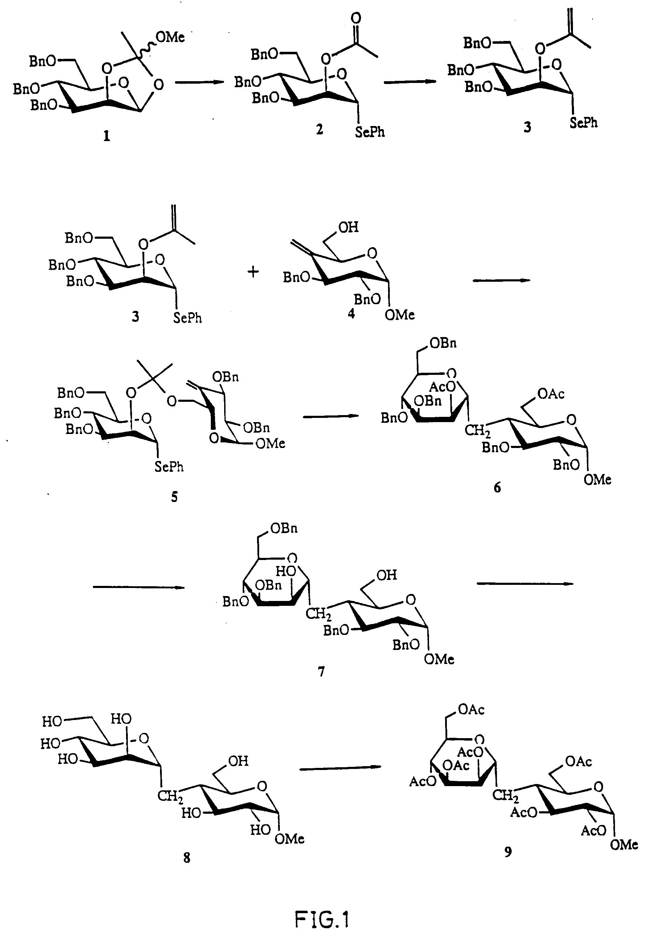

Fig. 1 represents the synthesis of a C-disaccharide using a ketal tether.

### DETAILED DESCRIPTION OF THE PREFERRED EMBODIMENTS OF THE INVENTION

More particularly, the present invention relates to the synthesis of C-disaccharides via a 9 endo-trig-cyclization process. In this process a glycosyl donor is tethered with a glycosyl acceptor which after cyclization and detethering form C-disaccharides. A variety of glycosyl donors can be utilized as a starting compound in the present invention such as seleno-phenyl glycosides, thiophenyl glycosides, glycosyl halides, mercaptobenzothiazol glycosides, xanthates and the like. Moreover, the glycosyl acceptor utilized in the present invention includes any glycosyl compound with an exomethylene group attached thereto or an exo-difluoromethylene group.

By glycosyl donor is meant that a glycosyl derivative has a donor group attached thereto. This donor group is capable of generating a radical at the C-1 position on the ring moiety and therefore is a nucleophilic anomeric radical donor. By glycosyl acceptor is meant that a glycosyl derivative has an acceptor group which acceptor group is capable of accepting a radical to form a carbon-carbon bond.

Unlike the donor group, the acceptor group can be varied at any position on the starting glycosyl compound or derivative thereof, utilized including positions C-1 through C-6. An hydroxy group is also present on the ring moiety of the glycosyl acceptor. The hydroxy group selected for the connection may be at any position of the glycosyl acceptor.

By use of the term "tethering" is meant to fasten or restrain within a set radius; a connection. In the present invention a ketal group is used to temporarily tether the two starting compounds and thus facilitate a carbon-carbon bond formation. In the past, the intermolecular reaction of a nucleophilic anomeric radical with an alkene was used to synthesize C-glycosides and C-disaccharides. See, for example Giese et al. Angew Chem., Int. Ed. Engl., 22, 622 (1983); Adlington et al. J. Chem. Soc. Chem. Commun., 944, (1984); Giese et al. Angew Chem., Int. Ed. Engl., 25, 450 (1986); Giese et al. Tetrahedron Lett., 29, 1375 (1988) and Bimwala and Vogel J.Org. Chem., 57 2076 (1992). Although a silaketal tether has been reported to occur via a 7, 8 or 9 endo-trig mode of cyclization as described by Hutchinson et al. Tetrahedron Lett., 32, 573 (1991), the substrates utilized are very simple and the yield is rather low in the use of the 9-endo-trig process.

The process of the present invention is accomplished by use of the starting compounds described above. A preferred embodiment of the present invention is the synthesis of phenyl 2-O-(methyl vinyl)-3,4,6-tri-O-benzyl-1-seleno-α-D-mannopyranoside. The synthesis of this glycosyl donor can be preferably achieved by the treatment of a solution of 3,4,6-tri-O-benzyl-1,2-O(methoxy ethyl)-β-D-mannopyranose and selenophenol, for example. The selenophenol group may be substituted, for example with a group such as a thiophenol, halides, a mercaptobenzothiazol, xanthates and the like. Generally between 450 mg to 550 mg of the above-mentioned mannopyranose is utilized. It is preferable to use 500 mg. The selenophenol group is added in a quantity between 250 µl to 360 µl or from about 2.5 to about 3.5, preferably 3 equivalents. It is preferable to add the selenophenol in anhydrous acetonitrile under an argon atmosphere. To this mixture is further added between 65 mg to 75 mg, preferably 70 mg or 0.2 equivalents of mercury (II) bromide. After approximately 30 minutes, the reaction mixture is then diluted in dichloromethane, washed with a solution of soda, then by a solution of ammonium chloride, dried under magnesium sulfate and concentrated. The residue is then purified to flash chromatography on a silica column.

To form the vinyl ether of phenyl 2-O-acetyl-3,4,6-tri-O-benzyl-1-seleno-α-D-mannopyranoside, between 10-14 ml, preferably 12 ml of a mixture of tetrahydrofuran and pyridine (5:1) is added to this mannopyranoside and the reaction is cooled to between about -35°C to -45°C, preferably -40°C under an atmosphere of argon. Once the reaction has cooled to the desired temperature, a solution of 0.25 M to 0.75 M, preferably 0.5 M of Tebbe's reagent (µ-chlorobis (η⁵-2,4-cyclopentadien-1-yl)(dimethylaluminum)-µ-methylene titanium) in anhydrous toluene, approximately 6.25 ml or 1.5 equivalents is then added. After mixing between 2.5 to 12 minutes, preferably 5 minutes, the reaction mixture is allowed to reach ambient temperature. Once ambient temperature has been reached, the reaction mixture is then cooled once again to between -15°C to -25°C, preferably -20°C and mixed with a 20% solution of soda. Approximately 2 ml of soda is added. The temperature is then brought to ambient temperature after the soda is added and filtered on celite and eluted with ether. The residue is then purified by flash chromatography on a column of silica. The vinyl ether is thus obtained as the glycosyl donor.

Once the glycosyl donor has been synthesized, it can be reacted with the glycosyl acceptor in order to form an intermediate ketal tether group. The different glycosyl acceptors that can be utilized in the present invention were discussed above. A preferable embodiment of the present invention uses an exomethylene sugar as the acceptor group. Therefore, a derivative of a 4-exo methylene derivative of methyl 2,3-di-O-benzyl-α-D-glucopyranoside such as methyl 2,3-di-O-benzyl-4-deoxy-4-C-methylene-α-D-xylo-hexopyranoside can be used in the present invention. The intermediate ketal tether is synthesized by mixing the glycosyl donor, glycosyl acceptor and any water absorbing non-reactive material such as a powdered molecular sieve, to absorb, when necessary, the remaining water in the reaction mixture. Between 750 mg to 850 mg, preferably 800 mg of the molecular sieve is utilized. In the preferred embodiment of the present invention, methyl 2,3-di-O-benzyl-4-deoxy-4-C-methylene-α-D-xylo-hexopyranoside is mixed with phenyl 2-O-(methyl ethyl)-3,4,6-tri-O-benzyl-1-seleno-α-D-mannopyranoside and anhydrous acetonitrile. The amounts of glycosyl donor and glycosyl acceptor utilized may vary; usually between about 450 mg to 550 mg of the glycosyl donor is used, preferably 500 mg and 285 mg to 310 mg of glycosyl acceptor or preferably 1 equivalent is used.

After mixing the above constituents, the reaction mixture is then cooled to about -40°C and a solution of between about 0.95 ml to 1.3 ml, preferably 1.1 ml of a solvent including paratoluene sulfonic acid, trifluoromethanesulfonic acid, camphorsulfonic acid and the like, preferably camphorsulfonic acid in acetonitrile (10 g/l) is added. The reaction is allowed to react for 40 to 50, preferably 45 minutes at -40°C. After incubation, approximately 100 µl anhydrous of any aminated base such as diisoproylethyldiamine, triethylamine and the like, preferably anhydrous triethylamine is added and the temperature is allowed to come to ambient temperature. The solution is filtered over silica and eluted with ether. The residue is then purified by flash chromatography over a silica column. The ketal tethered intermediate is then formed.

The tethered intermediate is then cyclized after being dissolved in a solvent such as benzene, xylene toluene and the like and refluxed. It is preferable to use anhydrous degassed toluene as the solvent. Any method of cyclization can be utilized in the present invention. For example, such cyclization methods using tributylin hydride, tris(trimethylsilyl)silane with any radical initiator such as benzoyl peroxide, triethylborane and the like or even photochemistry may be utilized. However it is preferable to cyclize the tethered ketal intermediate with a solution of tributylin hydride and azobisisobutyronitrile which is added over a period of 15 to 25 hours, preferably 17 hours. The tributylin hydride is present in an amount of between 150 to 225 µl, preferably 180 µl, in about 3 to 6 mg, preferably 5 mg, of azobisisobutyronitrile in a solvents such as benzene, xylene, toluene and the like. It is preferable to degass the solvent prior to use. Depending on the solvent and the initiator utilized, the temperature in the cyclization reaction may vary from -78°C to 140°C, for example.

After evaporating the residue to dryness, the residue is then further purified by flash chromatography over silica. The obtained mixture is then dissolved in a solvent such as chloroform, methanol, dichloromethane and the like and mixed with between 5 µl to 30 µl, preferably 20 µl of an acid such as acetic acid, sulfonic acid, trifluoroacetic acid and the like, and about 20 µl of distilled water. It is preferable to use dichloromethane and trifluoroacetic acid. After about 15 minutes, between 40 µl to 80 µl, preferably 60 µl of an aminated base such as diisopropyldiamine, triethylamine and the like, preferably triethylamine, is added and the solution is then concentrated. The residue obtained is then purified further by flash chromatography over silica gel.

After cyclization and detethering, a mixture of two C-disaccharides is obtained. To obtain methyl-6-O-acetyl-2,3-di-O-benzyl-4-O-(2-O-acetyl-3,4, 6-tri-O-benzyl-α-D-mannopyranosyl-methyl)-α-D-glucopyranoside, between 0.5 ml to 1.5 ml, preferably 1.0 ml of acetic anhydride or acetyl chloride is then added to the detethered mixture in pyridine, triethylamine:dichloromethane, for example. The present invention also includes the use of any other acetylating agents under conditions known in the art for acetylating. After incubation overnight, the reaction mixture is then concentrated and the residue is purified via flash chromatography over a silica column.

The acetyl intermediate may be then converted to a diol intermediate by addition of sodium methylate in methanol, neutralization by amberlite resin, filtering, concentrating this mixture and purifying via flash chromatography over silica. Between 30 mg to 50 mg, preferably 40 mg of methyl 6-O-acetyl-2,3-di-O-benzyl-4-O-(2-O-acetyl-3,4,6-tri-O-benzyl-α-D-mannopyranosyl- methyl)-α-D-glucopyranoside is suspended in about 0.6 M of sodium methylate in about 1 ml of methanol. Thus, methyl 2,3-di-O-benzyl-4-O-(3,4,6-tri-O-benzyl-α-D-mannopyranosyl methyl)-α-D-glucopyranoside is obtained.

Debenzylation of the methyl 2,3-di-O-benzyl-4-O-(3,4,6-tri-O-benzyl-α-D-mannopyranosyl methyl)-α-D-glucopyranoside to yield the methyl-C-disaccharide occurs by adding to this compound a mixture of a solvent such as ethanol, propanol, ethyl acetate, methanol and the like, between 5% to 12%, preferably 10% palladium over charcoal, and acetic acid if necessary. After this addition, the mixture is placed over a hydrogen atmosphere by a series of successive purges and mixed for at least 20 minutes. The reaction mixture is then filtered and concentrated.

The methyl 2,3,6-tri-O-acetyl-4-O-(2,3,4,6-tetra-O-acetyl-α-D-mannopyranosyl-methyl)-α-D-glucopyranoside can be synthesized by dissolving the methyl C-disaccharide in a mixture of pyridine:acetic anhydride (2:1) and reacting overnight. Between 6.0 mg to 8.0 mg, preferably 7.1 mg of the above-mentioned glucopyranoside and approximately 250 µl to 350 µl, preferably 300 µl of pyridine: acetic anhydride is used. The reaction mixture can then be purified by flash chromatography on a silica column.

Figure 1 illustrates a preferred embodiment of the process of present invention using the preferred starting compounds and preferred conditions.

In the following examples, the melting points were determined by a Buchi 510 apparatus and were not corrected. The optical rotation was measured with a Perkin-Elmer 241 polarimeter. The spectras of mass ionization chemical/ammonia were obtained by a Nermag R10-10 spectrophotometer. The NMR spectras of protons and carbon 13 were registered by Cameca 250 or Bruker AM-400 spectrophotometers with SiMe₄ as an internal reference and CDCl₃ as a solvent. The flash chromatographies were performed using silica gel 60(230-400 mesh, Merck). The microanalysis was performed by the service of microanalysis at the University of Jussieu-Paris.

In order to further illustrate the present invention and the advantages thereof, the following specific examples are given, it being understood that same are intended only as illustrative and in nowise limitative.

### EXAMPLE 1

### A. Synthesis of Compound 2-Phenyl-2-O-acetyl-3,4,6-tri-O-benzyl-1-seleno-α-D-mannopyranoside

70 Milligrams of mercury (II) bromide was added to a solution of 3,4,6-tri-O-benzyl-1,2- O-(methoxyethyl)-β-D-mannopyranose (500 mg of Compound 1) and 310 µl of selenophenol (3 equivalents) in anhydrous acetonitrile (15 ml) under an atmosphere of argon. After 30 minutes the reaction mixture was diluted in dichloromethane, washed with a solution of soda, then with a solution of ammonium chloride, and afterwards dried over magnesium sulfate and concentrated. The residue was purified by flash chromatography on a column of silica. 546 Milligrams of Compound 2 was obtained in the form of yellow white crystals. (87%)
m.p. 65-66°C (ether,hexane)
[α]_{D}+117 (c 1,0, CHCl₃)
NMR ¹H (250 MHz, CDCl₃)

| δ: | | | | |
|---|---|---|---|---|
| 7,62-7,21 | m | 20H | | H arom. |
| 5,84 | d | 1H | J_{1,2}1 Hz | H-1 |
| 5,71 | dd | 1H | J_{2,3}3 Hz | H-2 |
| 4,92 & 4,71 | 2d | 2H | J10,8 Hz | OCH₂Ph |
| 4,76 & 4,55 | 2d | 2H | J11,3 Hz | OCH₂Ph |
| 4,60 & 4,50 | 2d | 2H | J11,9 Hz | OCH₂Ph |
| 4,26 | ddd | 1H | J_{4,5}9,3;J_{5,6a}4,2;J_{5,6b}1,5 Hz | H-5 |
| 4,03 | dd | 1H | J_{3,4}9,3 Hz | H-4 |
| 3,94 | dd | 1H | | H-3 |
| 3,91 | dd | 1H | J_{6a,6b}11 Hz | H-6a |
| 3,75 | dd | 1H | | H-6b |
| 2,17 | s | 3H | | COCH₃ |

### B. Synthesis of Compound 3-Phenyl-2-O-(methyl-vinyl)-3,4,6-tri-O-benzyl-1-seleno-α-D-mannopyranoside

1 Gram of Compound 2 was dissolved in a mixture of tetrahydrofuran (THF):anhydrous pyridine (5:1, 12 ml). The solution was then cooled to -40°C, and a solution of 0.5 M of Tebbe's reagent in anhydrous toluene (1.5 equivalents) was added under an argon atmosphere. After 5 minutes of mixing at -40°C the temperature was allowed to reach ambient temperature. After 30 minutes the reaction mixture was recooled to -20°C and then a solution of 20% aqueous soda (2 ml) was added. The reaction mixture was then allowed to reach ambient temperature, then filtered on celite and eluted with ether. The residue was then purified by flash chromatography on a column of silica. 877 Milligrams of Compound 3 (87%) was obtained in the form of white crystals.
m.p. 62.5-63.5°C (ether, hexane)
[α]_{D}+114(c 1,0, CHCl₃)
NMR ¹H (250 MHz, C₆D₆)

| δ: | | | | |
|---|---|---|---|---|
| 7,66-6,92 | m | 20H | | H arom. |
| 6,31 | d | 1H | J_{1,2}1 Hz | H-1 |
| 5,00 & 4,59 | 2d | 2H | J11,3 Hz | OCH₂Ph |
| 4,82 | dd | 1H | J_{2,3}3,3 Hz | H-3 |
| 4,47 & 4,37 | 2d | 2H | J12,2 Hz | OCH₂Ph |
| 4,46 & 4,29 | 2d | 2H | J11,5 Hz | OCH₂Ph |
| 4,370 | ddd | 1H | J_{4,5}9,8;J_{5,6a}4,7;J_{5,6b}1 Hz | H-5 |
| 4,36 | dd | 1H | J_{3,4}9,1 Hz | H-4 |
| 4,07 | dd | 1H | | H-3 |
| 3,82 | dd | 1H | J_{6a,6b}10,9 Hz | H-6a |
| 3,82 & 3,80 | 2s | 2H | | C=CH₂ |
| 3,68 | dd | 1H | | H-6b |
| 1,75 | s | 3H | | CH₃ |

### C. Synthesis of Compound 5

To a flask containing Compound 3 (500 mg), Compound 4 (295 mg, 1 equivalent) and a powdered molecular sieve (4Å, 800 mg) a 25 ml solution of anhydrous acetonitrile was added. This solution was cooled to -40°C and a solution of camphorsulfonic acid in acetonitrile (10 g/l, 1.1 ml) was added under an argon atmosphere. After 45 minutes at -40°C, 100 µl of anhydrous triethylamine was added and the temperature was allowed to reach ambient temperature. After the temperature reached ambient temperature, the solution was then filtered on silica and eluted with ether. The residue was purified by flash chromatography on a column of silica. 652 Milligrams of Compound 5 (82%) in the form of white crystals was obtained.
m.p. 93-94°C (ether, hexane)
[α]_{D}+130 (c 0,94, CHCl₃)

### D. Cyclization of the radical of Compound 5

Composition 5 (300 mg) was dissolved in 7.5 ml of degassed anhydrous toluene and the solution was refluxed in toluene. A solution of tributylin hydride (180 µl) and azobisisobutyronitrile (5 mg) in 10 ml of degassed anhydrous toluene was added to Compound 5 with the aid of a syringe over a period of 17 hours. After evaporation to dryness, the residue was purified by flash chromatography on a column of silica. The mixture obtained was added to 3 ml of dichloromethane. To this solution was added 20 µl of trifluoroacetic acid and 20 µl of distilled water. After 15 minutes, 60 µl of triethylamine was added and the solution was then concentrated. The residue was then purified by flash chromatography over a column of silica. A mixture (10:1) of two C-disaccharides (92 mg, 38%) in the form of white syrup was obtained.

### E. Synthesis of Compound-6-methyl 6-O-acetyl-2, 3-di-O-benzyl-4-O-(2-O-acetyl-3,4,6-tri-O-benzyl-α-D-mannopyranosyl-methyl)-α-D-glucopyranoside

The preceding mixture obtained in part D was dissolved in 2 ml of pyridine and 1 ml of acetic anhydride was added. After one night the reaction mixture was then concentrated. The residue was then purified by flash chromatography over a silica column. 80 Milligrams of Compound 6 (79%) in the form of white crystals was obtained.
m.p. 102-103°C (ether, hexane)
[α]_{D}+12 (c 0,83, CHCl₃)

### F. Synthesis of Compound 7-Methyl 2,3-di-O-benzyl-4-O-(3, 4, 6-tri-O-benzyl-α-D-mannopyranosyl-methyl)-α-D-glucopyranoside

40 Milligrams of a suspension of Compound 6 in a solution sodium methylate in 0.06 M of methanol (1 ml) was mixed for 4 hours. The mixture was neutralized by amberlite resin (IR-120 H⁺) filtered and then concentrated. The residue was then purified by flash chromatography on a column of silica. 33 Milligrams of Compound 7 (92%) in the form of a white syrup was obtained.
[α]_{D}+41 (c 0,86, CHCl₃)

### G. Synthesis of Compound 8-Methyl 4-O-(α-D-mannopyranosyl-methyl)-α-D-glucopyranoside

Compound 7 (33 mg) was dissolved in a mixture of methanol:acetic acid (10:1, 11 ml). To this mixture was added via a spatula 10% palladium on carbon. The mixture was then placed under a hydrogen atmosphere by a series of successive purges and then mixed for 48 hours. The reaction mixture was then filtered and concentrated. 13.3 Milligrams of Compound 8 (92%) was obtained.
[α]_{D}+79 (c 0,26, CH₃OH)

### H. Synthesis of Compound 9-Methyl 2,3,6-tri-O-acetyl-4-O-(2,3,4,6-tetra-O-acetyl-α-D-mannopyranosyl-methyl)-α-D-glucopyranoside

7.1 Milligrams of Compound 8 was dissolved in a mixture of pyridine:acetic anhydride (2:1, 300 µl). After one night the reaction mixture was concentrated and then purified by flash chromatography over a column of silica. 8.6 Milligrams of Compound 9 (66%) in the form of a yellow syrup was obtained.
[α]_{D}+64 (c 0,86, CHCl₃)
NMR ¹H (400 MHz, CDCl₃)

| δ: | | | | |
|---|---|---|---|---|
| 5,39 | dd | 1H | J_{2,3}10;J_{3,4}10,5 Hz | H-3 |
| 5,22 | dd | 1H | J_{2',3'}3,5;J_{3',4'}6,5 Hz | H-3' |
| 5,03 | dd | 1H | J_{4',5'}1 Hz | H-4' |
| 4,94 | dd | 1H | J_{1',2'}6,5 Hz | H-2' |
| 4,92 | d | 1H | J_{1,2}3,5 Hz | H-1 |
| 4,81 | dd | 1H | | H-2 |
| 4,50 | dd | 1H | 7_{5'6'a}7,5;J_{6'a,6'b}12 Hz | H-6'a |
| 4,37 | dd | 1H | J_{5,6a}2;J_{6a,6b}12 Hz | H-6a |
| 4,15 | dd | 1H | J_{5,6b}5,5 Hz | H-6b |
| 4,14 | dd | 1H | J_{5',6'b}4,5 Hz | H-6'b |
| 3,95 | ddd | 1H | | H-5' |
| 3,90 | ddd | 1H | J_{1'αa}=J_{1'αb}6,5 Hz | H-1' |
| 3,81 | ddd | 1H | J_{4,5}11 Hz | H-5 |
| 3,40 | s | 3H | | OCH₃ |
| 2,15-2,07 | 7s | 21H | | 7COCH₃ |
| 2,05-1,94 | m | 1H | | H-4 |
| 1,64-1,58 | m | 2H | | H-αa,αb |

## Claims

1. A process for the synthesis of C-disaccharides comprising the steps of:
(a) synthesizing a glycosyl donor wherein said glycosyl donor contains a vinyl ether attached to a glycosyl moiety;
(b) condensing said glycosyl donor with a glycosyl acceptor having an exomethylene group attached to a glycosyl moiety on said glycosyl acceptor to form a temporary ketal;
(c) cyclizing said temporary ketal;
(d) detethering said cyclized temporary ketal to form a protected C-disaccharide; and
(e) debenzylating said protected C-disaccharide to form a methyl α-C-disaccharide.

2. The process according to Claim 1, wherein said glycosyl donor is phenyl 2-O-(methyl-vinyl)-3,4,6-tri-benzyl-1-seleno-α-D-mannopyranoside.

3. The process according to Claim 1 or 2, wherein the glycosyl acceptor is an 4-exo methylene derivative of methyl 2,3-di-O-benzyl-α-D-glucopyranoside.

4. The process according to Claims 1 to 3, wherein the glycosyl acceptor is methyl 2,3-di-O-benzyl-4-deoxy-4-C-methylene-α-D-xylo-hexopyranoside.

5. The process according to any one of Claims 1 to 4, wherein the ketal intermediate is a compound having the following formula:

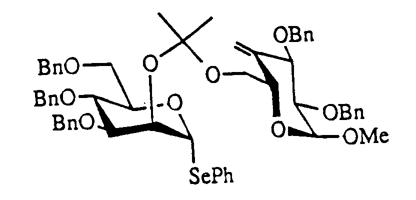

6. The process according to any one of Claims 1 to 5, wherein after cyclization in step (c) and detethering in step (d), acetylating said cyclized tethered intermediate to form methyl 6-O-acetyl-2,3-di-O-benzyl-4-O-(2-O-acetyl-3,4,6-tri-O-benzyl-α-D-mannopyranosyl-methyl)-α-D-glucopyranoside.

7. The process according to Claim 1, wherein said methyl α-C-disaccharide is methyl-4-O-(α-D -mannopyranosyl-methyl)-α-D-glucopyranoside.

8. The process according to Claim 1, further comprising the optional step of after debenzylating of reacting said C-disaccharide with a mixture of pyridine:acetic anhydride to form methyl 2,3,6-tri-O-acetyl-4-O-(2,3,4,6-tetra-O-acetyl-α-O- mannopyranosyl-methyl)-α-D-glucopyranoside.

9. The process according to Claim 1, wherein said glycosyl donor is synthesized by treating a solution of 3,4,6-tri-O-benzyl-1,2-O-(methoxy ethyl)-β-D-mannopyranose and selenophenol with mercury (II) bromide to form phenyl 2-O-acetyl-3,4,6-tri-O-benzyl-1-seleno-α-D-mannopyranoside and converting said phenyl 2-O-acetyl-3,4,6-tri-O-benzyl-1-seleno-α-D-mannopyranoside to phenyl 2-O-(methyl-vinyl)-3,4,6-tri-O-benzyl-1-seleno-α-D-mannopyranoside via addition of tetrahydrofuran and pyridine, followed by Tebbe's reagent.

10. A process for the synthesis of a C-disaccharide comprising the steps of:
(a) synthesizing a glycosyl donor of phenyl 2-O-(methyl-vinyl)-3,4,6-tri-O-benzyl-1-seleno-α-D-mannopyranoside from 3,4,6-tri-O-benzyl-1, 2-0-(methoxy ethyl)-β-D-mannopyranose;
(b) condensing said glycosyl donor of phenyl-2-O-(methyl-vinyl)-3,4,6-tri-O-benzyl-1-seleno-α-D-mannopyranoside with a solution of methyl-2,3-di-O-benzyl-4-deoxy-4-6-methylene-α-D-xylo-hexopyranoside to form a tethered ketal intermediate;
(c) cyclizing said tethered ketal intermediate;
(d) detethering and acetylating said cyclized tethered ketal intermediate to form methyl 6-O-acetyl-2,3-di-O-benzyl-4-O-(2-O-acetyl-3,4,6-tri-O-benzyl-α-D-mannopyranosyl-methyl)-α-D- glucopyranoside; and
(e) de-acetylating and debenzylating methyl 6-O-acetyl-2,3-di-O-benzyl-4-O-(2-O-acetyl-3, 4,6-tri-O-benzyl-α-D-mannopyranosyl-methyl)-α -D-glucopyranoside to form methyl 4-O-(α-D-mannopyranosyl-methyl)-α-D-glucopyranoside.
